(19) Europäisches Patentamt
European Patent Office
Office européen des brevets

(11) Veröffentlichungsnummer: **0 431 451 B1**

(12) **EUROPÄISCHE PATENTSCHRIFT**

(45) Veröffentlichungstag der Patentschrift: **06.04.94**

(51) Int. Cl.5: **C07C 209/60**

(21) Anmeldenummer: **90122698.5**

(22) Anmeldetag: **28.11.90**

(54) **Verfahren zur Herstellung von Aminen.**

(30) Priorität: **06.12.89 DE 3940349**

(43) Veröffentlichungstag der Anmeldung:
**12.06.91 Patentblatt 91/24**

(45) Bekanntmachung des Hinweises auf die Patenterteilung:
**06.04.94 Patentblatt 94/14**

(84) Benannte Vertragsstaaten:
**BE CH DE FR GB IT LI NL**

(56) Entgegenhaltungen:
**EP-A- 0 039 918**
**EP-A- 0 132 736**
**EP-A- 0 133 938**
**EP-A- 0 263 462**

(73) Patentinhaber: **BASF Aktiengesellschaft**
**Carl-Bosch-Strasse 38**
**D-67063 Ludwigshafen(DE)**

(72) Erfinder: **Hesse, Michael, Dr.**
**An der Froschlache 3**
**W-6700 Ludwigshafen(DE)**
Erfinder: **Steck, Werner. Dr.**
**Auerstrasse 4**
**W-6700 Ludwigshafen(DE)**
Erfinder: **Lermer, Helmut, Dr.**
**Bockenheimer Strasse 12**
**W-6700 Ludwigshafen(DE)**
Erfinder: **Fischer, Roman, Dr.**
**Deidesheimer Strasse 1**
**W-6704 Mutterstadt(DE)**
Erfinder: **Schwarzmann, Matthias, Dr.**
**Carl-Bosch-Strasse 54**
**W-6703 Limburgerhof(DE)**

**Beschreibung**

Die Erfindung betrifft ein Verfahren zur Herstellung von Aminen durch Anlagerung von Ammoniak oder primären oder sekundären Aminen an Olefine in Gegenwart von Galliumsilikat-Zeolithen des Pentasil-Typs.

Nach den bekannten Verfahren zur Addition von Ammoniak oder primären oder sekundären Aminen an Olefine werden nur ungenügende Selektivitäten und/oder Katalysator-Standzeiten erreicht, wie beispielsweise nach US-A 2 623 061, US-A 2 422 631, US-A 2 501 556 und US-A 3 412 158. Auch das in US-A 4 307 250 beschriebene Verfahren eignet sich nicht für die Durchführung im technischen Maßstab. Die hier als Katalysator verwendeten Aluminium-Zeolithe begünstigen die Polymerbildung und nachfolgende Verkokung, die den Katalysator desaktiviert.

Aus der DE-A-33 27 000 und der DE-A-33 26 579 ist bekannt, daß sich bestimmte Bor-, Aluminium- und Eisensilikatzeolithe für die Herstellung von Aminen aus Olefinen gut eignen. Aus der DE-A-36 34 247 ist die Möglichkeit der Dotierung dieser Zeolithe mit übergangsmetallen ebenfalls bekannt. Diese Katalysatoren waren in ihren Standzeiten und/oder Selektivitäten noch verbesserungsbedürftig.

Der vorliegenden Erfindung lag daher die Aufgabe zugrunde, den zuvor genannten Nachteilen abzuhelfen.

Demgemäß wurde gefunden, daß man hohe Standzeiten und hohe Selektivitäten der Katalysatoren bei der Herstellung von Aminen aus Olefinen und Ammoniak oder primären oder sekundären Aminen bei Temperaturen zwischen 80°C und 400°C und bei Drücken zwischen 40 und 700 bar in Gegenwart eines zeolithischen Katalysators erzielt, wenn man in Gegenwart eines Galliumsilikat-Zeolithen des Pentasil-Typs als Katalysator umsetzt.

Durch den Einsatz dieser Galliumsilikat-Zeolithe vom Pentasiltyp werden z.B. an einem undotierten Katalysator gleiche Umsätze, jedoch überraschenderweise höhere Selektivitäten und höhere Standzeiten erreicht als in den Fällen der DE-A-33 27 000, der DE-A-33 26 579 und der DE-A-36 34 247. Es kann somit gegenüber den oben genannten Verfahren auf eine übergangsmetalldotierung wie beispielsweise mit Cr verzichtet werden, ohne merkliche Ausbeuteeinbußen hinnehmen zu müssen. Dadurch wird die Katalysatorherstellung um mehrere Verfahrensstufen verringert und Umweltprobleme, die bei der Entsorgung oder Aufarbeitung von schwermetallhaltigen Katalysatoren entstehen könnten a priori vermieden.

Das erfindungsgemäße Verfahren zeichnet sich dadurch aus, daß bereits mit niedrigem Ammoniak- bzw. Amin-überschuß eine hohe Selektivität an gewünschtem Reaktionsprodukt erzielt und die Dimerisierung und/oder Oligomerisierung des eingesetzten Olefins vermieden wird. Gleichzeitig werden hohe Standzeiten beobachtet.

Eine Ausführungsform dieses Verfahrens besteht darin, daß man Ammoniak und/oder Amine zusammen mit Olefinen im molaren Verhältnis von 0,8:1 bis 50:1 vorzugsweise 1:1 bis 5:1 gemischt einem Festbett- oder Wirbelbettreaktor zuführt und bei einem Druck von 40 bis 700 bar, insbesondere 200 bis 300 bar und einer Temperatur von 80 bis 400 °C, insbesondere 250 bis 350 °C in der Gasphase oder im überkritischen Zustand umsetzt. Eine andere Ausführungsform besteht darin, daß die Reaktion in Flüssigphase bei einem Druck von 40 bis 80 bar und einer Temperatur von 60 °C bis 120 °C in einem Rührkessel, einem Fest-/Flüssigkeits-Fließbett oder einem Strömungsrohr durchgeführt wird.

Aus dem Reaktionsaustrag wird das gewünschte Produkt mit Hilfe bekannter Methoden, beispielsweise Destillation oder Extraktion, erhalten und nötigenfalls mittels weiterer Trennoperationen auf die gewünschte Reinheit gebracht. Die nichtumgesetzten Eingangsstoffe werden in den Reaktor zurückgeführt.

Man kann einfach oder mehrfach ungesättigte Olefine mit 2 bis 10 C-Atomen bzw. deren Mischungen als Ausgangsstoffe verwenden. Wegen der geringer ausgeprägten Polymerisationsneigung eignen sich Monoolefine besser als Di- und Polyolefine, doch können diese mit Hilfe höherer Ammoniak- bzw. Aminüberschüsse ebenso selektiv umgesetzt werden. Gut geeignete Olefine sind beispielsweise Ethen, Propen, 1-Buten, 2-Buten, 1-Penten, 2-Methylpropen, 2-Methylbutene, 1-Hexene, Cyclopenten und Cyclohexen. Als primäre Amine eignen sich Alkylamine mit 1 bis 10 C-Atomen wie Methylamin, Ethylamin, Propylamin, Isopropylamin und Cyclohexylamin oder Anilin, als sekundäre Amine eignen sich Dialkylamine mit 2 bis 20 C-Atomen wie Dimethylamin, Diethylamin und Methylethylamin oder Alkyl- und Phenylenthaltende Amine wie Methyl-Phenylamin oder Diphenylamin.

Bevorzugte Aminendprodukte sind: Ethylamin, Isopropylamin, Cyclohexylamin, Cyclopentylamin und tert.-Butylamin.

Die Lage des Gleichgewichts und damit der Umsatz zum gewünschten Amin ist sehr stark vom gewählten Reaktionsdruck abhängig. Hoher Druck begünstigt das Additionsprodukt, doch stellt im allgemeinen aus technischen und wirtschaftlichen Gründen der Druckbereich bis 300 bar das Optimum dar. Die Selektivität der Reaktion wird - neben Größen wie Ammoniak-/Amin-überschuß und Katalysator - in hohem Maß durch die Temperatur beeinflußt. Zwar nimmt die Reaktionsgeschwindigkeit der Additionsreaktion mit steigender Temperatur stark zu, doch werden konkurrierende Crack- und Rekombinationsreaktionen des Olefins gleichzeitig gefördert. Die Lage des Temperaturoptimums bezüglich Umsatz und Selektivität ist von der Konstitution des Olefins des eingesetzten Amins und des Katalysators abhängig und liegt meist im Bereich von 250 bis 350 °C. Die Verweilzeit ist abhängig von den Einsatzstoffen und liegt zweckmäßigerweise im Bereich zwischen Bruchteilen einer Sekunde und wenigen Minuten.

Als Katalysatoren für die Aminierung von Olefinen verwendet man Galliumsilikat-Zeolithe des Pentasiltyps, insbesondere solche der Formel $M_{2/n}O*Ga_2O_3*ySiO_2$, in der M Wasserstoff oder ein Alkali- oder Erdalkalimetall und n die Wertigkeit von M ist, bevorzugt M Wasserstoff und n die Zahl 1 bedeuten. Besonders gut eignen sich Ga-Pentasile, die ein molares Verhältnis von $SiO_2/Ga_2O_3$ zwischen 20 und 800 aufweisen und zumindest teilweise in der H-Form vorliegen.

Der Galliumsilikatzeolith des Pentasiltyps wird z. B. bei 90 bis 200 °C unter autogenem Druck synthetisiert, indem man eine Galliumverbindung, z.B. ein Alkaligallat, bevorzugt Natriumgallat, oder ein Galliumoxid oder Galliumhalogenid oder andere geeignete Galliumsalze , mit einer Siliciumverbindung, z. B. Alkalisilikaten, Kieselsolen, Kieselsäureestern oder vorzugsweise hochdispersem Siliciumdioxid in wäßriger Aminlösung, z.B. primären, sekundären oder tertiären Aminen oder quarternären Alkylammoniumverbindungen, wobei ein oder mehrere Aminfunktionen pro Molekül vorliegen können, z.B. in 1,6-Diaminohexan-Lösung oder insbesondere Tetrapropylammoniumhydroxidlösung, mit oder ohne Alkali- oder Erdalkalizusatz zur Reaktion bringt. Die Herstellung von Zeolithen in Gegenwart dieser Amine ist beispielsweise beschrieben in US-A 3 702 886, BE-A 886 833, BE-A 882 484 und DE-AS 30 06 471.

Die so hergestellten Galliumsilikatzeolithe können nach ihrer Isolierung, Trocknung bei 100 bis 160 °C, vorzugsweise 110 °C und Calcinierung bei 400 bis 600 °C, vorzugsweise 500 °C, mit einem Bindemittel im Verhältnis 90 : 10 bis 40 : 60 Gew.-% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 95 : 5, bevorzugt 75 : 25, Siliciumdioxid, bevorzugt hochdisperses $SiO_2$, Gemische aus hochdispersem $SiO_2$ und hochdispersem $Al_2O_3$, $TiO_2$, bevorzugt hochdisperses $TiO_2$, $ZrO_2$ sowie Ton. Nach der Verformung werden die Extrudate oder Preßlinge bei 110 °C/16 h getrocknet und bei 500 °C/16 h calciniert.

Die Röntgen-Diffraktogramme zum Nachweis der Pentasilstruktur wurden nach den üblichen Methoden aufgenommen. Die Lagen der Netzebenenabstände entsprechen den für ZSM-5 in US-A 3 702 886 und für ZSM-11 in US-A-3 709 979 mitgeteilten Werten.

Man erhält auch vorteilhafte Katalysatoren, wenn der isolierte Galliumsilikatzeolith direkt nach der Trocknung verformt und erst nach der Verformung einer Calcinierung unterworfen wird. Die hergestellten Galliumsilikatzeolithe können in reiner Form, d.h. ohne Binder, als Stränge oder Tabletten eingesetzt werden, wobei als Verstrangungs- oder Peptisierungshilfsmittel z. B. Ethylcellulose, Stearinsäure, Kartoffelstärke, Ameisensäure, Oxalsäure, Essigsäure, Salpetersäure, Ammoniak, Amine und Graphit oder deren Gemische verwendet werden.

Liegt der Zeolith aufgrund der Art seiner Herstellung nicht in der katalytisch aktiven, aciden H-Form vor, sondern z. B. in der Na-Form, dann kann er gemäß dem Stand der Technik durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren evtl. eine durch Koksabscheidung bedingte Desaktivierung eintritt, empfiehlt es sich, die Zeolithe durch Abbrennen der Koksablagerung mit Luft oder mit einem Luft/-$N_2$-Gemisch bei 400 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Die hier beschriebenen Katalysatoren können wahlweise als 2 bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Schwerflüchtige oder feste Edukte werden in gelöster Form z. B. in THF-, Toluol- oder Petrolether-Lösung zum Einsatz gebracht. Generell ist eine Verdünnung des Eduktes mit derartigen Lösungsmitteln oder mit Inertgasen wie $N_2$, Ar, He, $H_2O$-Dampf möglich.

Die folgenden Beispiele veranschaulichen die Erfindung.

Beispiele 1 - 5 und Vergleichsbeispiele 1 und 2

Die Reaktionen wurden unter isothermen Bedingungen in einem Rohrreaktor (Wendel, 0,6 cm Innendurchmesser, 90 cm Länge) durchgeführt. Die Temperatur betrug 300 °C, der Druck 300 bar. Die

EP 0 431 451 B1

Reaktionsprodukte wurden durch übliche Methoden abgetrennt und charakterisiert. Die quantitative Bestimmung der Reaktionsprodukte und der Ausgangsprodukte erfolgte gaschromatographisch.

Die für das erfindungsgemäße Verfahren eingesetzten Katalysatoren sind:

Katalysator A:

167 g wäßrige Tetra-n-propylammoniumhydroxid-Lösung (20 Gew.-%) wird in eine Suspension aus 1 600 g Wasser und 100 g pyrogener Kieselsäure eingerührt. Dann wird eine Lösung aus 4,4 g Natriumhydroxid, 67 g Wasser und 41 g Natriumgallat-Lösung (12,2 Gew.-% Na, 12,7 Gew.-% $Ga_2O_3$) hinzugefügt. Die Reaktionsmischung wird 96 Stunden bei 175 °C unter autogenem Druck erhitzt.

Nach Abfiltrieren und Auswaschen wird das kristalline Reaktionsprodukt bei 110 °C/24 h getrocknet und bei 500 °C/24 h calciniert. Dieses Galliumsilikatpentasil enthält 81.7 Gew.-% SiO2 und 4.6 Gew.-% $Ga_2O_3$.

Mit diesem Material werden durch Verformen mit einem Verformungshilfsmittel 2-mm-Stränge hergestellt, die bei 110 °C/16 h getrocknet und bei 500 °C/24 h calciniert werden.

Die Stränge werden mit 20%iger NH4Cl-Lösung (Massenverhältnis 1 : 15) bei 80 °C ausgetauscht. Dann wird Chlorid-frei gewaschen, bei 110 °C getrocknet und 5 h bei 500 °C calciniert. Der Na-Gehalt beträgt 0,003 %.

Katalysator B:

Katalysator B wird hergestellt, indem ein Ga-Pentasil gemäß Katalysator 4 in der Pulverform mit $SiO_2$ - (Gewichtsverhältnis 80 : 20) zu 2-mm-Strängen verstrangt wird, die bei 110 °C/16 h getrocknet und bei 500 °C/16 h calciniert werden.

Die Stränge werden mit 20%iger $NH_4$Cl-Lösung (Massenverhältnis 1 : 15) bei 80 °C ausgetauscht. Dann wird Chlorid-frei gewaschen, bei 110 °C getrocknet und 5 h bei 500 °C calciniert. Der Na-Gehalt beträgt 0,003%

Katalysator C:

Katalysator C wird hergestellt, indem ein Ga-Pentasil gemäß Katalysator A in der Pulverform mit Boehmit (Gewichtsverhältnis 60:40) zu 2-mm-Strängen verformt wird, die bei 110 °C/16 h getrocknet und bei 500 °C/16 h calciniert werden.

Die Stränge werden mit 20%iger $NH_4$Cl-Lösung (Massenverhältnis 1 : 15) bei 80 °C ausgetauscht. Dann wird Chlorid-frei gewaschen, bei 110 °C getrocknet und 5 h bei 500 °C calciniert. Der Na-Gehalt beträgt 0,009%.

Katalysator D:

Katalysator D ist ein Aluminiumsilikat-Zeolith, hergestellt unter hydro-thermalen Bedingungen bei autogenem Druck und 150 °C aus 65 g $SiO_2$ (hochdispers) und 20,3 g $Al_2(SO_4)3$ x 18 $H_2O$ in 1 kg einer wäßrigen 1,6-Diamino-hexan-Lösung (Mischung 50 : 50 Gew.-%) in einem Rührautoklaven. Nach Abfiltrieren und Auswaschen wird das kristalline Reaktionsprodukt bei 110 °C/24 h getrocknet und bei 500 °C/24 h calciniert. Dieser Aluminiumsilikat-Zeolith enthält 91,6 Gew.-% $SiO_2$ und 4,6 Gew.-% $Al_2O_3$. Dieses Produkt wird mit Boehmit im Gewichtsverhältnis 60 : 40 zu 2-mm-Strängen verformt und bei 110 °C/16 h getrocknet und bei 500 °C/24 h calciniert.

Katalysator E:

Katalysator E wird durch Tränkung von 50 g Katalysator D mit 9,39 g $Zn(NO_3)2$ x 6 $H_2O$ - in 20 g Wasser gelöst - hergestellt. Nach dem Tränken wird bei 130 °C/2 h getrocknet und bei 540 °C/2 h calciniert.

Beispiele 1 bis 5

Die Reaktionen wurden unter isothermen Bedingungen in einem Rohrreaktor (Wendel, 0,6 cm Innendurchmesser, 90 cm Länge) durchgeführt. Die Temperatur betrug 300 °C, der Druck 300 bar. Die Reaktionsprodukte wurden durch übliche Methoden abgetrennt und charakterisiert. Die quantitative Bestimmung der Reaktionsprodukte und der Ausgangsprodukte erfolgte gaschromatographisch.

4

Die Ergebnisse sind in den Tabellen 1 und 2 zusammengestellt:

Tabelle 1

| Beispiel | 1 | 2 | 3 |
|---|---|---|---|
| Katalysator | A | B | C |
| Umsatz von Isobuten | 15.2 % | 14.7 % | 14.5 % |
| Selektivität tert.-Butylamin | > 98.0 % | > 98.0 % | > 98.0 % |

Tabelle 2

| Vergleichsbeispiel | 4 | 5 |
|---|---|---|
| Katalysator | D | E |
| Umsatz von Isobuten | 11.1 % | 12.1 % |
| Selektivität tert.-Butylamin | 93.6 % | 95.2 % |

Beispiel 6 und 7

Für eine kontinuierliche Herstellung wird der oben beschriebene HochdruckReaktor eingesetzt. Ein Gemisch aus Isobuten und Ammoniak (1 : 1.3 molar) wird bei 300 °C, 300 bar und einer Belastung von 4 kg/l Katalysator und Stunde umgesetzt. Katalysator A (Beispiel 6) und B (Beispiel 7) zeigen nach zweiwöchiger Laufzeit unveränderte Aktivität und Selektivität.

**Patentansprüche**

1. Verfahren zur Herstellung von Aminen aus Olefinen und Ammoniak oder primären oder sekundären Aminen bei Temperaturen zwischen 60°C und 400°C und Drücken zwischen 40 und 700 bar in Gegenwart eines zeolithischen Katalysators, dadurch gekennzeichnet, daß man die Umsetzung in Gegenwart eines Galliumsilikat-Zeolithen des PentasilTyps durchführt.

2. Verfahren nach Anspruch 1, dadurch gekennzeichnet, daß man Olefin und Ammoniak oder primäre oder sekundäre Amine oder Gemische derselben in Gegenwart eines Galliumsilikat-Zeolithen des Pentasil-Typs als Katalysator umsetzt, das erhaltene Amin abtrennt und die nichtum-gesetzten Einsatzstoffe zurückführt.

3. Verfahren nach Anspruch 1, dadurch gekennzeichnet, daß man als zeolithischen Katalysator ein Galliumsilikat-Zeolith des Pentasiltyps der Formel $M_{2/n}O*Ga_2O_3*ySio_2$ verwendet, in der M Wasserstoff oder ein Alkali- oder Erdalkalimetall und n die Wertigkeit von M ist, bedeutet und y Werte von 20 bis 800 hat.

4. Verfahren nach Anspruch 1, dadurch gekennzeichnet, daß man als zeolithischen Katalysator ein Galliumsilikat-Zeolith des Pentasiltyps der Formel $M_{2/n}O*Ga_2O_3*ySiO_2$ verwendet, in der M Wasserstoff bedeutet, n für 1 steht und y Werte` von 20 bis 800 hat.

5

**Claims**

1. A process for the preparation of an amine from olefin and ammonia or a primary or secondary amine at from 60 to 400 °C and from 40 to 700 bar in the presence of a zeolite catalyst, wherein the reaction is carried out in the presence of a gallium silicate zeolite of the pentasil type.

2. A process as claimed in claim 1, wherein an olefin and ammonia or a primary or secondary amine or a mixture thereof is reacted in the presence of a gallium silicate zeolite of the pentasil type as a catalyst, the amine obtained is separated off and the unconverted starting materials are recycled.

3. A process as claimed in claim 1, wherein the zeolite catalyst used is a gallium silicate zeolite of the pentasil type of the formula $M_{2/n}O.Ga_2O_3.ySiO_2$, where M is hydrogen or an alkali metal or alkaline earth metal, n is the valency of M and y is from 20 to 800.

4. A process as claimed in claim 1, wherein the zeolite catalyst used is a gallium silicate zeolite of the pentasil type of the formula $M_{2/n}O.Ga_2O_3.ySiO_2$, where M is hydrogen, n is 1 and y is from 20 to 800.

**Revendications**

1. Procédé de préparation d'amines à partir d'oléfines et d'ammoniac, ou d'amines primaires ou secondaires, à des températures comprises entre 60 °C et 400 °C et sous des pressions comprises entre 40 et 700 bar, en présence d'un catalyseur zéolithique, caractérisé en ce que l'on effectue la réaction en présence d'une zéolithe de silicate de gallium du type pentasil.

2. Procédé selon la revendication 1, caractérisé en ce que l'on fait réagir une oléfine et de l'ammoniac, ou une amine primaire ou secondaire, ou des mélanges de ceux-ci, en présence d'une zéolithe de silicate de gallium du type pentasil comme catalyseur, l'on sépare l'amine obtenue et l'on recycle les réactifs n'ayant pas réagi.

3. Procédé selon la revendication 1, caractérisé en ce que l'on utilise comme catalyseur zéolithique une zéolithe de silicate de gallium du type pentasil de formule $M_{2/n}O*Ga_2O_3*ySiO_2$, dans laquelle M est l'hydrogène ou un métal alcalin ou alcalino-terreux et n la valence de M, y ayant une valeur comprise entre 20 et 800.

4. Procédé selon la revendication 1, caractérisé en ce que l'on utilise comme catalyseur zéolithique une zéolithe de silicate de gallium du type pentasil de formule $M_{2/n}O*Ga_2O_3*ySiO_2$, dans laquelle M représente l'hydrogène, n est égal a 1 et y a une valeur comprise entre 20 et 800.